# EUROPEAN PATENT SPECIFICATION

(11) **EP 2 068 760 B1**
(45) Date of publication and mention of the grant of the patent: **05.11.2014**
(21) Application number: 07811450.1
(22) Date of filing: 21.08.2007
(51) Int. Cl.: A61F 2/02, A61L 31/04, A61L 31/06, A61L 31/10, A61L 31/14, A61L 31/16, A61F 2/00

(54) **DEVICE FOR TISSUE REINFORCEMENT HAVING MICROPOROUS AND MACROPOROUS STRUCTURES**
VORRICHTUNG ZUR GEWEBEVERSTÄRKUNG MIT MIKROPORÖSEN UND MAKROPORÖSEN STRUKTUREN
DISPOSITIF PERMETTANT LE RENFORCEMENT D'UN TISSU QUI POSSÈDE DES STRUCTURES MICROPOREUSE ET MACROPOREUSE

(30) Priority: 29.09.2006 US 540826
(43) Date of publication of application: 17.06.2009
(73) Proprietor: ETHICON, INC., West Somerville, NJ 08876 (US)
(72) Inventor: YANG, Chunlin, Belle Mead, NJ 08502 (US); KAMMERER, Gene, W., East Brunswick, NJ 08816 (US); ARNOLD, Kelly, R., Sparta, NJ 07871 (US); DO, Hiep, Sinking Spring, PA 19608 (US)
(74) Representative: Kirsch, Susan Edith
(86) International application number: PCT/US2007/018449
(87) International publication number: WO 2008/042057

(56) References cited:
- US-A- 5 743 917
- US-A1- 2003 149 440
- US-B1- 6 306 424
- US-B2- 6 599 323
- US-B2- 6 599 323
- Bernd Klosterhalfen ET AL: "The lightweight and large porous mesh concept for hernia repair", Expert Rev. Med. Devices, 1 January 2005 (2005-01-01), pages 1-15, XP55040014, DOI: 10.1586/17434440.2.1.xxx Retrieved from the Internet: URL:http://www.ethicon.de/netze/downloads/ Lightweight_mesh_Klosterhafen.pdf [retrieved on 2012-10-04]
- S. TRUONG ET AL: 'Results after endoscopic treatment of postoperative upper gastrointestinal fistulas and leaks using combined Vicryl plug and fibrin glue' SURGICAL ENDOSCOPY vol. 18, no. 7, 01 July 2004, XP055068888 DOI: 10.1007/s00464-003-8286-7 ISSN: 0930-2794

## Description

### FIELD OF THE INVENTION

The present invention relates to devices for tissue reinforcement, more specifically devices having both a macroporous and microporous structure to allow both cell in-growth and tissue integration.

### BACKGROUND OF THE INVENTION

Individuals can sometimes sustain an injury to tissue (for example, trauma, stress induced, overuse, etc.), such as musculoskeletal tissue, that requires repair by surgical intervention. There are numerous surgical procedures in which a supportive or reconstructive technique is used to repair the injured tissue within the body. In many cases surgical sutures are employed to strengthen, support and repair weak tissue in these procedures. The sutures are especially useful in the repair of ligaments, muscles and fascia layers, which hold organs in place. However, in some cases the simple repair by re-attachment or tying layers of tissue together is not appropriate to provide a satisfactory outcome. In that case it may be required to replace some of these supportive tissues. There are then standard accepted techniques which surgeons use as well as accepted materials. Examples the accepted materials, which are inserted into the body to repair and replace damaged supportive tissue include, xenografts, a surgical graft of tissue from one species onto or into individuals of unlike species, genus or family. This is also known as a heteroplastic graft. Allografts, grafts between two or more individuals allogenic at one or more loci (usually with reference to histocompatibility loci. Autografts, grafts taken from one part of the body and placed in another site on the same individual, usually harvested at the time of the procedure. Synthetic fabrics constructed in the configuration of knits, weaves and expanded foams, using biocompatible materials some of which absorb in the body and some which do not and remain permanently in place.

One example of a fairly common tissue injury in females is damage to the pelvic floor. This is a potentially serious medical condition that may occur, for example, during childbirth or from complications thereof, which can result in an injury of the vesicovaginal fascia. Such an injury can result in a cystocele, which is a herniation of the bladder. Similar medical conditions include rectoceles (a herniation of the rectum), enteroceles (a protrusion of the intestine through the rectovaginal or vesicovaginal pouch), and enterocystoceles (a double hernia in which both the bladder and intestine protrude). These conditions can be serious medical problems that can severely and negatively impact a patient both physicologically and psychologically.

These conditions are typically treated by surgical procedures in which the protruding organs or portions thereof are repositioned. A mesh-like patch is often used to repair the site of the protrusion.

Various known devices and techniques for treating such conditions have been described in the prior art. For example, it is known to use an intravaginal set, i.e., surgical mesh sold under the tradename PROLIFT (Johnson & Johnson Corporation, New Brunswick NJ) a medical device used to contract the pelvic floor muscles and elevate the pelvic floor.

In addition, it is known to use a biocompatible repair patch having a plurality of apertures formed therein, which is formed of woven, knitted, non-knitted, or braided biocompatible polymers. Such a patch can be coated with a variety of bioabsorbable materials as well as another material that can decrease the possibility of infection, and/or increase biocompatibility.

Other known reinforcing materials include biopolymer foams and foam constructs that can be used in tissue repair and reconstruction, and an open cell, foam-like implant made from resorbable materials, which has one or more textile reinforcing elements embedded therein.

Another known biocompatible tissue implant has a reinforcement material, preferably a mesh, and a bioabsorbable polymeric foam having pores with an open cell structure.

US-B-6599323 describes reinforced tissue implants comprising a bioabsorbable foam reinforced with mesh material. The foam completely fills the pores of the reinforcing mesh in order to interlock the foam on the opposite sides of the mesh.

Klosterhalfen et al. in Expert Rev. Med. Devices vol. 2(1) pages 1-15 (2005) describe a comparative study of a number of different meshes for hernia repair. Meshes comprising both absorbable and non-absorbable elements are mentioned. Lightweight meshes having large pores are suggested as most suitable for hernia repair.

US-A-20030149440 describes mesh strips for use in surgical methods of treating female urinary incontinence. The mesh strips may comprise a natural material placed over or incorporated within a generally central portion of a synthetic mesh strip.

US-B-6306424 describes bioabsorbable composites comprising a fibrous bioabsorbable layer laminated to a continuous, three-dimensional foam layer

Despite existing technology, there continues to be a need for a bioabsorbable tissue repair implant having sufficient structural integrity to withstand the stresses associated with implantation into an affected area and also possesses capability to not only promote tissue in growth but also enhance the integration of implants with ingrown tissues.

### SUMMARY OF THE INVENTION

The present invention provides a tissue reinforcement device comprising: a macroporous structure in which the pore size is greater than 200µm; and a microporous material having a pore size of from 5µm to 150µm, characterized in that said microporous material is in the form of a coating having a thickness of from 10µm to 500µm applied to at least a part of the macroporous structure without filling the pores of said part of the macroporous structure.

The devices of the present invention may be incorporated with bioactives and therapeutic cells. The devices of the present invention have numerous advantages including enhanced tissue repair, facilitating tissue regeneration, eliminating or reducing potential scar contraction and formation, preventing or reducing the incidence of infection, and/or to preventing tissue adhesion.

These and other aspects and advantages of the present invention will become more apparent from the following description and accompanying drawings.

### BRIEF DESCRIPTION OF THE FIGURES

FIG. 1 is an SEM Image (37 X) of a tissue reinforcement device having a polypropylene mesh macrostructure wherein the individual polypropylene fibers are coated with 36/64 PCL/PGA microporous foam.
FIG. 2 is an SEM Image (350 X) of the tissue reinforcement device of FIG. 1 having a polypropylene mesh macrostructure where the individual polypropylene fibers are coated with 36/64 PCL/PGA microporous foam.

### DETAILED DESCRIPTION OF THE INVENTION

As used herein, the term "microporous coating" is defined to be a coating in which the pores sizes range between 5 microns and 150 microns. A "macroporus structure" is defined to be a structure in which the pore size is greater than 200 microns. The macroporus structure can be constructed from a variety of conventional, biocompatible materials including biodegradable, non-biodegradable, and natural polymeric materials. The optional individual components of the macroporous structure are preferably fibers or a bundle of fibers. The macroporous structure may have a variety of constructs including a flat sheet, a rolled sheet, a hollow structure, a solid structure, and a 3-dimensional structure, is preferably in the form of a knitted or woven mesh. If desired, radio opacity may be added to the construct by rendering at least a portion of the macroporous component radioopaque. This can be achieved by weaving in a radioopaque fibrous component to mark different regions of the macroporous structure.

The individual components of the macroporous structure of the tissue implant of the present invention can be comprised of any biodegradable or non-biodegradable biocompatible material, including textiles with woven, knitted, warped knitted (i.e., lace-like), non-woven, and braided structures. In an exemplary embodiment the macroporous structure has a mesh-like structure. In any of the above structures, the mechanical properties of the structure can be altered by changing the density or texture of the material, or by embedding particles in the material. Fibers used to make the individual components of a macroporous mesh-like structure can be monofilaments, yarns, threads, braids, or bundles of fibers. These fibers can be made of any biocompatible material including biodegradable or non-biodegradable materials.

Non-biodegradable materials include cotton, linen, silk, polyamides, polyesters, fluoropolymers, polyolefins and combinations thereof.

Biodegradable polymers readily break down into small segments when exposed to moist body tissue. The segments then either are absorbed by the body, or passed by the body. More particularly, the biodegraded segments do not elicit permanent chronic foreign body reaction, because they are absorbed by the body or passed from the body, such that no permanent trace or residual of the segment is retained by the body. Biodegradable materials include polymers such as polylactic acid (PLA), polyglycolic acid (PGA), polycaprolactone (PCL), polydioxanone (PDO), trimethylene carbonate (TMC), polyvinyl alcohol (PVA), copolymers or blends thereof. In one embodiment, the fibers are formed of a polylactic acid and polyglycolic acid copolymer at a 95:5 mole ratio.

In another embodiment, the fibers that form the individual components of a macroporous structure having a mesh-like construct can be made of a biocompatible, biodegradable glass. Bioglass, a silicate containing calcium phosphate glass, or calcium phosphate glass with varying amounts of ionic substitutions for calcium added to control resorption time are examples of materials that could be spun into glass fibers and used for the individual components of the macroporous structure. Suitable ions that may be substituted for calcium include iron, magnesium, sodium, potassium, and combinations thereof.

The individual components of the macroporous structure are coated with a microporous coating. The microporous coating is preferably in the form of a biodegradable foam. The microporous coating of the tissue implant device may be formed and/or applied in a variety of conventional processes including lyophilization, supercritical solvent foaming, gas injection extrusion, gas injection molding or casting with an extractable material, such as salts, sugar or similar suitable materials, preferably by lyophilization.

The microporous coating may also be applied by other conventional processes such as dipping, injecting, pouring, or otherwise placing, the appropriate polymer solution into a mold set-up comprised of a mold and the macroporous structure. Care must be taken to coat the macroporous structure to prevent the microporous coating from filling the pores of the macroporous structure.

The mold set-up is cooled in an appropriate bath or on a refrigerated shelf and then lyophilized. In the course of forming the foam coating, it is believed to be important to control the rate of freezing of the polymer-solvent system. The type of pore morphology that is developed during the freezing step is a function of factors such as the solution thermodynamics, freezing rate, temperature to which it is cooled, concentration of the solution, and whether homogeneous or heterogenous nucleation occurs. One of ordinary skill in the art can readily optimize the parameters without undue experimentation.

The steps involved in the preparation of these foams include choosing the right solvents for the polymers to be lyophilized and preparing a homogeneous solution. Next, the polymer solution is subjected to a freezing and vacuum drying cycle. The freezing step phase separates the polymer solution and vacuum drying step removes the solvent by sublimation and/or drying, leaving a microporous polymer structure.

Suitable solvents that may be used in the preparation of the foam coating include, but are not limited to, formic acid, ethyl formate, acetic acid, hexafluoroisopropanol (HFIP), cyclic ethers (e.g., tetrahydrofuran (THF), dimethylene fluoride (DMF), and polydioxanone (PDO), acetone, acetates of C2 to C5 alcohols (e.g., ethyl acetate and t-butylacetate), glyme (e.g., monoglyme, ethyl glyme, diglyme, ethyl diglyme, triglyme, butyl diglyme and tetraglyme), methylethyl ketone, dipropyleneglycol methyl ether, lactones (e.g., gamma-valerolactone, delta-valerolactone, beta-butyrolactone, γ-butyrolactone), 1,4-dioxane, 1, 3-dioxolane, 1,3-dioxolane-2-one(ethylene carbonate), dimethlycarbonate, benzene, toluene, benzyl alcohol, p-xylene, naphthalene, tetrahydrofuran, N-methyl pyrrolidone, dimethylformamide, chloroform, 1,2-dichloromethane, morpholine, dimethylsulfoxide, hexafluoroacetone sesquihydrate (HFAS), anisole and mixtures thereof. Among these solvents, a preferred solvent is 1,4-dioxane. A homogeneous solution of the polymer in the solvent is prepared using standard techniques.

The applicable polymer concentration or amount of solvent that may be utilized will vary with each system. Generally, the amount of polymer in the solution can vary from about 0.5% to about 90% and, preferably, will vary from about 0.5% to about 30% by weight, depending on factors such as the solubility of the polymer in a given solvent and the final properties desired in the foam.

Various conventional biocompatible, biodegradable polymers can be used for the microporous coatings of the tissue reinforcement devices according to the present invention. Examples of suitable biocompatible, bioabsorbable polymers include polymers selected from the group consisting of aliphatic polyesters, poly(amino acids), copoly(ether-esters), polyalkylenes oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, biomolecules (i.e., biopolymers such as collagen, elastin, bioabsorbable starches, etc.), collagen-glycosoaminoglycan (GAG), and blends thereof.

Currently, aliphatic polyesters are among the preferred absorbable polymers for use in making the microporous coatings according to the present invention. Aliphatic polyesters can be homopolymers, copolymers (random, block, segmented, tappered blocks, graft, triblock, etc.) having a linear, branched or star structure. Suitable monomers for making aliphatic homopolymers and copolymers may be selected from the group consisting of, but are not limited, to lactic acid, lactide (including L-, D-, meso and D,L mixtures), glycolic acid, glycolide, epsilon-caprolactone, p-dioxanone (1,4-dioxan-2-one), trimethylene carbonate (1,3-dioxan-2-one), and combinations thereof.

Biocompatible, elastomeric copolymers are also particularly useful in the practice of the present invention. Suitable elastomeric polymers include those with an inherent viscosity in the range of about 1.2 dL/g to 4 dL/g, more preferably about 1.2 dL/g to 2 dL/g and most preferably about 1.4 dL/g to 2 dL/g as determined at 25° C. in a 0.1 gram per deciliter (g/dL) solution of polymer in hexafluoroisopropanol (HFIP). Further, suitable elastomers exhibit a high percent elongation and a low modulus, while possessing good tensile strength and good recovery characteristics. In the preferred embodiments of this invention, the elastomer from which the microporous component is formed exhibits a percent elongation (e.g., greater than about 200 percent and preferably greater than about 500 percent). In addition to these elongation and modulus properties, suitable elastomers should also have a tensile strength greater than about 3.4 MPa (500 psi), preferably greater than about 6.9 MPa (1,000 psi), and a tear strength of greater than about 8.8 k'N/m (50 lbs/inch), preferably greater than about 14 k'N/m (80 lbs/inch).

Exemplary bioabsorbable, biocompatible elastomers include but are not limited to elastomeric copolymers of epsilon-caprolactone and glycolide (including polyglycolic acid) with a mole ratio of epsilon-caprolactone to glycolide of from about 35:65 to about 65:35, more preferably from 35:65 to 45:55; elastomeric copolymers of epsilon-caprolactone and lactide (including L-lactide, D-lactide, blends thereof, and lactic acid polymers and copolymers) where the mole ratio of epsilon-caprolactone to lactide is from about 30:70 or to about 95:5; elastomeric copolymers of p-dioxanone (1,4-dioxan-2-one) and lactide (including L-lactide, D-lactide, blends thereof, and lactic acid polymers and copolymers) where the mole ratio of p-dioxanone to lactide is from about 40:60 to about 60:40; elastomeric copolymers of epsilon-caprolactone and p-dioxanone where the mole ratio of epsilon-caprolactone to p-dioxanone is from about from 30:70 to about 70:30; elastomeric copolymers of p-dioxanone and trimethylene carbonate where the mole ratio of p-dioxanone to trimethylene carbonate is from about 30:70 to about 70:30; elastomeric copolymers of trimethylene carbonate and glycolide (including polyglycolic acid) where the mole ratio of trimethylene carbonate to glycolide is from about 30:70 to about 70:30; elastomeric copolymers of trimethylene carbonate and lactide (including L- lactide, D-lactide, blends thereof, and lactic acid polymers and copolymers) where the mole ratio of trimethylene carbonate to lactide is from about 30:70 to about 70:30); and blends thereof.

One of ordinary skill in the art will appreciate that the selection of a suitable polymer or copolymer for forming the microporous coating depends on several factors. The more relevant factors in the selection of the appropriate polymer(s) that is used to form the coating include bioabsorption (or bio-degradation) kinetics; in vivo mechanical performance; cell response to the material in terms of cell attachment, proliferation, migration and differentiation; and biocompatibility. Other relevant factors, which to some extent dictate the in vitro and in vivo behavior of the polymer, include the chemical composition, spatial distribution of the constituents, the molecular weight of the polymer, and the degree of crystallinity.

The thickness of the coating is in the range of 10 to 500 microns, preferably 50 to 500 microns. The microporous coating has pores sizes in the range of 5 to 150 microns, preferably 10 to 100 microns. This pore size is sufficient effective to encourage the attachment and proliferation of cells.

The size of the device must be sufficiently large to effectively cover at least a section of the defect and may vary in size based on the size of the defect. In a flat sheet embodiment, for example, the size of the device in one application may be about 24 centimeters by 24 centimeters and can be fashioned into various shapes (square, rectangle, trapezoid, etc.), based on the configuration of the defect, and the size will vary accordingly.

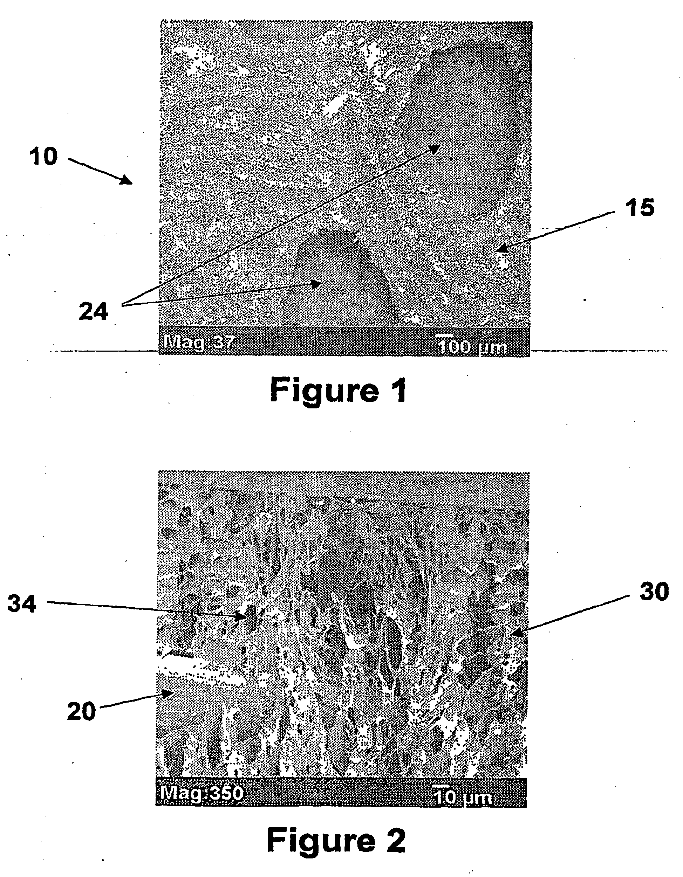

FIGS. 1 and 2 show an embodiment of the present invention. The tissue reinforcement device 10 is seen to have individual components 20 that make up the macroporous structure 15 of the device 10. The components 20 in this embodiment are polypropylene fibers. Components 20 are coated with foam coating 30. Device 10 has macropores 24 in macroporous structure 15 and micropores 34 in coating 30.

The location and coverage of the microporous coating on the macroporous structure will vary. In some embodiments, one or more sections of macroporous structure are coated with microporous structure. In order to accomplish this, portions or sections of the macroporous structure including optional individual components are dipped into the appropriate polymer solution, or the appropriate polymer solution is injected into, poured onto, or otherwise placed onto, portions of the macroporous structure and/or components.

In another embodiment, once the structure disclosed in the present invention is created, a secondary operation can be conducted to produce specific shapes or configurations to enhance the performance of the reinforcement. For example, in the case of a mesh like construct, the material can be utilized as a flat sheet, cut to desired shapes, rolled into a tube structure producing a design with various diameters, wall thicknesses and lengths, etc.

If desired, a sufficiently effective amount of a bioactive agent may be incorporated within and/or applied to the tissue reinforcement device, and/or it can be applied to the viable tissue at the point of care. Preferably, the bioactive agent is incorporated within, or coated on, the device prior to the addition of viable tissue to the device. The bioactive agent(s) can be selected from among a variety of conventional effectors that, when present at the site of injury, promote healing and/or regeneration of the affected tissue. In addition to being compounds or agents that actually promote or expedite healing, the effectors may also include compounds or agents that prevent infection (e.g., antimicrobial agents and antibiotics), compounds or agents that reduce inflammation (e.g., anti-inflammatory agents), compounds that prevent or minimize adhesion formation, such as oxidized regenerated cellulose (e.g., INTERCEDED® and Surgicel®, available from Ethicon, Inc.), hyaluronic acid, and compounds or agents that suppress the immune system (e.g., immunosuppressants).

One possible application of a tissue implant device of the present invention is as a matrix for use in herniation or pelvic floor repair surgical procedures. Herniation may include abdominal hernias, such as inguinal hernias, femoral hernias, umbilical hernias, and incisional hernias.

The term "pelvic floor" refers to the pelvic diaphragm, the sphincter mechanism of the lower urinary tract, the upper and lower vaginal supports, and the internal and external anal sphincters. It is a network of muscles, ligaments and other tissues that hold up the pelvic organs (vagina, rectum, uterus and bladder). When this system is torn or weakens, the organs may shift, bulge and push outward or against each other. A prolapse occurs when an organ drops from its natural position. As a result, women may suffer from urinary or fecal incontinence or obstruction, vaginal prolapse, vaginal pain, sexual dysfunction, and other problems. Women who vaginally delivered several children and those who experienced tears in the perineum and pelvic floor during childbirth, are at higher risk for pelvic floor disorders.

Additional factors contributing to pelvic floor relaxation include aging, menopause, connective tissue disorders, degenerative neurologic conditions, and prior pelvic surgery. Any of these factors alone or in combination may occur acutely or over time, and result in some of the most common and feared health problems faced by women.

In general, the method of treating a tissue injury using the tissue reinforcement device of the present invention comprises the steps of accessing the site of the damaged tissue, preparing the site for implant (i.e., device) placement, placing the tissue treatment device in a section of damaged tissue, securing the device to the site, and exiting the site of the damaged tissue. Access may be via incision or through laproscopy. The device may be secured by friction with the tissue. This can be accomplishes by sandwiching the device between layers of tissue. Alternatively, the device may be sutured in place.

For example, pelvic reconstructive surgery can be performed through the vagina or abdominally via traditional incision or through laproscopy. During the procedure, the surgeon will reposition the prolapsed organ and secure it to the surrounding tissues and ligaments. The tissue reinforcement device discussed herein is inserted into the region of the prolapse. It is initially held in place by friction or sutures. Body tissue then grows into the macropores of the device, creating the final support. The strength of this tissue is greatly enhanced by the presence of the device.

The various components in the device serve different functions. The macroporous structure encourages ingrowth into the device to be as rapid as possible, and provides for secure attachment to the area of application. The microporous coating or structure encourages the attachment and proliferation of cells. The structure can be optimized for cell infiltration by smooth muscle cells or fibroblasts, and for vascularization.

Examples of the types of specific procedures in which a device of this invention would be beneficial is the repair of the prolapse of the vagina by suspension from the sacral area. Sacrocolpopexy is attachment of the to the bone and sacrospinous fixation is attachment of the vagina to the sacrospinous ligament. This procedure is mostly done when the patient has previously or concomitantly had a hystorectomy. Alternately, the repair of a prolapse of the uterus into the vagina can be accomplished using a similar procedure again by suspension from the previously mentioned points in the sacral area. Since the uterus is still intact this procedure is a sacrohysteropexy.

The following examples are illustrative of the principles and practice of this invention.

### EXAMPLES

### Example 1: Formation of Tissue Reinforcement Device

A 10 weight percent solution of epsilon-caprolactone/glycolide copolymer in 1,4-dioxane was prepared as follows. 20.1 grams of 36/64 poly(epsilon-caprolactone-glycolic acid) copolymer (PCGA), obtained from American Polymer Incorporation (American Polymer Inc., Birmingham, AL), was added to 180 grams of 1,4-dioxane (Fisher Scientific, Raritan, NJ) in a 250 milliliter Erlenmeyer screwed cap flask. The mixture was stirred for 4 hours in 60°C water bath set on a temperature controlled heating plate. The polymer solution was filtered through an extra coarse thimble filter to remove any non-dissolved solids. This 10 weight percent solution was diluted to 5 weight percent by mixing 75.0 grams of 10 percent solution with 75.2 grams of 1,4-dioxane (Fisher Scientific, Raritan, NJ).

A 3 weight percent solution was prepared by diluting 18.1 grams of 5 weight percent solution with 12.2 grams of 1,4-dioxane (Fisher Scientific, Raritan, NJ).

A piece of 3 inch by 6 inch of polypropylene mesh sold under the tradename GYNEMESH* PS (Ethicon, Inc, Somerville, NJ) was securely placed between two 51 mm by 127 mm (2 inch by 5 inch) aluminum frames. The aluminum frame containing mesh was dipped in 3 weight percent solution and the excess solution was shaken off prior to placement on a pre-cooled (-17°C) shelf of a freeze-dryer (lypholyzation unit) (FTS Systems, Model TD3B2T5100, Stone Ridge, NY). The following freeze-drying cycle was the used:

| Temperature(°C) | Pressure (mtorr) | Hold time (min) |
|---|---|---|
| -17 | Ambient | 15 |
| -17 | Ambient | 60 |
| -5 | 100 | 60 |
| +5 | 20 | 60 |
| 20 | 20 | 60 |

After completion of the lyophilization cycle, the tissue reinforcement device was removed from the aluminum frame, and stored in dry conditions.

### Example 2: SEM evaluation

Samples of the tissue reinforcement device made in Example 1 were mounted on a microscope stud and coated with a thin layer of gold using a EMS 550 sputter coater. SEM analysis was performed using the JEOL JSM-5900LV SEM. The surfaces and cross-sectional areas were examined for each sample. FIGS. 1 and 2 shows tissue reinforcement device 10 where the individual components 20 of the macroporous structure 15 of the device 10 are polypropylene fibers. The fibers are coated with foam coating 30. Device 10 has macropores 24 and micropores 34 in coating 30.

### Example 3: Cell Attachment

Human fibroblasts were used for this experiment. Five samples of the tissue reinforcement device made in Example 1 were additionally coated with human fibroblast cells. As a control, five samples of polypropylene mesh were also coated with human fibroblast cells. The number of cells attached to the devices after overnight incubation were measured using a CyQuant kit from Molecular Probe Inc. A higher level of fluorescence indicates a higher level of cell attachment.

The tissue reinforcement device made in Example 1 had a fluorescence of 643.6 +/- 333.6, while the fluorescence of the polypropylene mesh control was 1'74.6 +/- 44.8. The mesh with individual fibers coated with polymer foam showed enhanced cell attachment.

### Example 4- Surgical Procedures

A patient is prepared in a conventional manner for a sacrocolpopexy procedure. After accessing the site, the device is connected between the vagina and the sacrum, replacing the support to the vagina that would have been provided by the uterosacral ligaments. During the operation, the doctor stitches one end of the device to the top of the vagina and the other end to a bone near the spine (called the sacrum or sacral bone). Alternately it can be stitched to the sacrospinous ligament, which connects the sacrum to the ischial spine. This procedure can be done abdominally, either through keyhole surgery (laparoscopy) or a larger cut just above the bikini line, laporotomy. It can also be done transvaginally, which is through the vagina without making any incision in the external skin of the patient. This later procedure is accomplished by opening the back or apex of the vagina. In any one of these techniques the procedure is the same. The device is stitched or fastened to the outer surface of the vaginal apex on one side using sutures. The opposite end of the device is then pulled to approximate the vagina near the sacrum or sacrospinous ligament. This position represents the true anatomical position of the vagina. The end of the device is then fixed to the bone or ligament using a suture or a surgical fastener. A second device is then attached to the opposite side of the vagina at the apex as well, adjusted for the correct length and attached in the same way to the bone or ligament. In the trans-vaginal technique the apex is then stitched closed. For the laparoscopic technique and the open technique the abdominal incisions are then closed.

In the second mentioned procedure the uterus is re-suspended.

Treatments that suspend rather than remove the uterus are recommended for women who want to keep their uterus or have children in the future. Procedures can be done either vaginally or abdominally. The procedure is called a sacrohysteropexy. This procedure uses the device to hold the uterus in place. The operation is done abdominally, either through a small (typically 15-cm) cut just above the pubic hairline or through keyhole surgery (laparoscopy), or trans-vaginally through the back of the vagina. The doctor attaches one end of the device to the cervix and top of the vagina using sutures and the other to a bone (sacrum or sacral bone) near the spine again using sutures or in some cases using surgical fasteners or anchors. Generally, a hard anchor is either screwed into the bone or wedged into a hole, which is drilled into the bone. Sutures attached to the anchor are then threaded through the device and adjusted to bring the device in contact with the bone and surrounding tissues. This portion of the procedure is repeated on the opposite side of the uterus. The device replaces the support to the uterus, which is normally provided by the uterosacral ligaments, the cardinal ligaments and the broad ligament.

The devices and the use of the devices of the present invention in surgical procedures have many advantages. The advantage of this type of device is that the small micropores can allow for the attachment of cells to the device. The macropores provide for growth of the cells into the device, and a secure attachment of the reinforcement material to the surrounding tissue. This can avoid encapsulation of the device. The thinness of the coating also allows the device to be more supple than other composite structures.

It will be apparent to those skilled in the art that various modifications and variations can be made in the present invention without departing from the scope of the following claims.

## Claims

1. A tissue reinforcement device comprising:
a macroporous structure in which the pore size is greater than 200 µm; and
a microporous material having a pore size of from 5 µm to 150 µm,
**characterized in that** said microporous material is in the form of a coating having a thickness of from 10µm to 500µm applied to at least a part of the macroporous structure.

2. The device of claim 1, wherein the structure comprises a construct selected from the group consisting of woven, knitted, warped knitted, non-woven, and braided.

3. The device of claim 1, wherein the macroporous structure comprises a material that is not biodegradable.

4. The device of claim 3, wherein the material is selected from the group consisting of cotton, linen, silk, polyamides, polyester fluoropolymers and polyolefins.

5. The device of claim 1 wherein the macroporous material comprises a biodegradable material.

6. The device of claim 5, wherein the biodegradable material comprises a material selected from the group consisting of polymers, copolymers and glasses.

7. The device of claim 1, wherein the coating comprises a biodegradable foam.

8. The device of claim7, wherein the foam comprises a polymer selected from the group consisting of polymers and copolymers.

9. The device of claim 8, wherein the polymer is selected from the group consisting of polylactic acid, polyglycolic acid, polycaprolactone, polydioxanone, trimethylene carbonate, and polyvinyl alcohol.

10. The device of claim 1, additionally comprising a bioactive agent.

11. The device of claim 10, wherein the bioactive agent additionally comprises a bioactive agent selected from the group consisting of antimicrobial agents, antibiotics, anti-inflammatory agents, oxidized regenerated cellulose, hyaluronic acid, and immunosuppressants.

## Patentansprüche

1. Vorrichtung zur Gewebeverstärkung, umfassend:
eine makroporöse Struktur mit einer Porenweite größer 200 µm und
ein mikroporöses Material mit einer Porenweite von 5 µm bis 150 µm,
**dadurch gekennzeichnet, dass** das mikroporöse Material als eine auf der makroporösen Struktur zumindest teilflächig aufgebrachte Beschichtung in einer Stärke von 10 µm bis 500 µm vorliegt.

2. Vorrichtung nach Anspruch 1, bei der es sich bei der Struktur um ein Gebilde handelt, welches aus der aus Geweben, Gestricken, Gewirken, Vliesstoffen und Geflechten bestehenden Gruppe ausgewählt ist.

3. Vorrichtung nach Anspruch 1, bei der es sich bei der makroporösen Struktur um ein nicht biologisch abbaubares Material handelt.

4. Vorrichtung nach Anspruch 3, bei der das Material aus der aus Baumwolle, Leinen, Seide, Polyamiden, Polyestern, Fluorpolymeren und Polyolefinen bestehenden Gruppe ausgewählt ist.

5. Vorrichtung nach Anspruch 1, bei der es sich bei dem makroporösen Material um ein biologisch abbaubares Material handelt.

6. Vorrichtung nach Anspruch 5, bei der es sich bei dem biologisch abbaubaren Material um ein aus der aus Polymeren, Copolymeren und Glasen bestehenden Gruppe ausgewähltes Material handelt.

7. Vorrichtung nach Anspruch 1, bei der es sich bei der Beschichtung um einen biologisch abbaubaren Schaumstoff handelt.

8. Vorrichtung nach Anspruch 7, bei der es sich bei dem Schaumstoff um ein aus der aus Polymeren und Copolymeren bestehenden Gruppe ausgewähltes Polymer handelt.

9. Vorrichtung nach Anspruch 8, bei der das Polymer aus der aus Polymilchsäure, Polyglykolsäure, Polycaprolacton, Polydioxanon, Trimethylencarbonat und Polyvinylalkohol bestehenden Gruppe ausgewählt ist.

10. Vorrichtung nach Anspruch 1, zusätzlich umfassend einen biologisch aktiven Wirkstoff.

11. Vorrichtung nach Anspruch 10, bei der der biologisch aktive Wirkstoff zusätzlich einen biologisch aktiven Wirkstoff umfasst, welcher aus der aus antimikrobiellen Wirkstoffen, Antibiotika, Antiphlogistika, oxidierter Regeneratcellulose, Hyaluronsäure und Immunosuppressiva bestehenden Gruppe ausgewählt ist.

## Revendications

1. Dispositif de renforcement de tissu, comprenant:
une structure macroporeuse dans laquelle la taille de pore est supérieure à 200 µm; et
une matière microporeuse dont la taille de pore est comprise entre 5 µm et 150 µm,
**caractérisé en ce que** ladite matière microporeuse se présente sous la forme d'un revêtement qui présente une épaisseur comprise entre 10 µm et 500 µm appliqué à au moins une partie de la structure macroporeuse.

2. Dispositif selon la revendication 1, dans lequel la structure comprend un aspect construit sélectionné dans le groupe composé de tissé, tricoté, tricoté ondulé, non tissé et tressé.

3. Dispositif selon la revendication 1, dans lequel la structure macroporeuse comprend une matière qui n'est pas biodégradable.

4. Dispositif selon la revendication 3, dans lequel la matière est sélectionnée dans le groupe composé du coton, du lin, de la soie, de polyamides, de fluoropolymères de polyester et de polyoléfines.

5. Dispositif selon la revendication 1, dans lequel la matière macroporeuse comprend une matière biodégradable.

6. Dispositif selon la revendication 5, dans lequel la matière biodégradable comprend une matière qui est sélectionnée dans le groupe composé de polymères de copolymères et de verres.

7. Dispositif selon la revendication 1, dans lequel le revêtement comprend une mousse biodégradable.

8. Dispositif selon la revendication 7, dans lequel la mousse comprend un polymère sélectionné dans le groupe composé de polymères et de copolymères.

9. Dispositif selon la revendication 8, dans lequel le polymère est sélectionné dans le groupe composé de l'acide polylactique, de l'acide polyglycolique, du polycaprolactone, du polydioxanone, du carbonate de triméthylène et de l'alcool polyvinylique.

10. Dispositif selon la revendication 1, comprenant en outre un agent bioactif.

11. Dispositif selon la revendication 10, dans lequel l'agent bioactif comprend en outre un agent bioactif qui est sélectionné dans le groupe composé des agents microbiens, des antibiotiques, des agents anti-inflammatoires, de la cellulose régénérée oxydée, de l'acide hyaluronique et des immunosuppresseurs.
